# EUROPEAN PATENT APPLICATION

(11) **EP 3 831 846 A1**
(43) Date of publication of application: **09.06.2021**
(21) Application number: 19843928.3
(22) Date of filing: 31.07.2019
(51) Int. Cl.: C07K 16/00, C07K 16/46, C12N 15/13, C12N 15/62

(54) **SUPER VERSATILE METHOD FOR IMPARTING NEW BINDING SPECIFICITY TO ANTIBODY**

(30) Priority: 31.07.2018 JP 2018144345
(71) Applicant: The University of Tokyo, Bunkyo-ku, Tokyo 113-8654 (JP); Osaka University, Suita-shi, Osaka 565-0871 (JP)
(72) Inventor: SUGA, Hiroaki, Tokyo 113-8654 (JP); TAKAGI, Junichi, Suita-shi, Osaka 565-0871 (JP)
(74) Representative: D Young & Co LLP
(86) International application number: PCT/JP2019/030081
(87) International publication number: WO 2020/027224

(57) **Abstract**

The purpose of the present invention is to provide an efficient and versatile method for granting an additional binding activity to an antibody by inserting a peptide sequence into the antibody. The present invention provides a method of granting a binding ability to a second target molecule to an antibody, comprising inserting an internal peptide sequence of a cyclic peptide into an Fc region of the antibody, wherein the cyclic peptide has the binding ability to the second target molecule different from a first target molecule to be recognized by the antibody.

## Description

### Technical Field

The present invention relates to a highly versatile method for granting a new binding specificity to an antibody, and the like.

### Background Art

Antibodies essentially have a function of specifically binding to only one antigen molecule as a target molecule. As an engineered antibody obtained by a protein engineering method, there is an antibody (multi-specific antibody: MsAb) capable of recognizing two or more respectively different antigen molecules.

Since MsAb is an antibody that simultaneously binds to two or more target molecules, it can be used as a drug that crosslinks heterologous cells, for example, cancer cells and lymphocytes and thereby exhibits anticancer effects. In addition, MsAb obtained by granting second binding specificity to an antibody having original first binding specificity can be accumulated on desired tissues or cells.

Furthermore, even in the case where two antibodies, for example, an immune checkpoint inhibitor and an apoptosis inducing antibody should essentially be used in combination, use of MsAb makes it possible to replace administration of two antibodies by administration of one antibody and it is expected to lead to cost reduction.

Thus, MsAb is regarded as very important in the development of antibody drugs.

As MsAb, a bispecific antibody (BsAb) has a combination of two antigen recognizing Fab regions respectively derived from different antibodies, while a technology called "Fcab" creates IgG type BsAb by embedding second binding specificity in a site other than the Fab regions (Non-Patent Document 1).

In Fcab, the two Fab regions are from the original antibodies, respectively, so that divalent binding similar to that of a wild type IgG antibody is guaranteed for the first binding specificity which the antibody originally has.

In Fcab, the second binding specificity is obtained by a method of randomizing specific loop structures of the Fc region and selecting from them a structure having affinity for the target molecule. In this case, the resulting antibody acquires amino acid mutation corresponding to 30 residues or less in total in a plurality of regions within the Fc region compared with the original IgG antibody. The second binding specificity is granted to the Fc region which is independent from the Fab region so that once a Fc sequence having a binding specificity can be obtained, there is a possibility of using it in combination with the (Fab) of a plurality of antibodies to create various BsAbs.

However, selection of a specific binder from the randomized loop library is achieved with markedly low success probability and therefore requires considerable trial and error. In addition, in many cases, it requires a step of, after stages including artificial molecular evolution based on a weak binder sequence, finding a binder having affinity enough for a practical level. Further, by such a method, only one binding specificity can usually be granted to an IgG antibody. There has been no example of succeeding in granting third and fourth binding specificities to the antibody.

There is disclosed a technology of making use of the Fc region of an antibody for a physiologically active peptide having usually low stability in a body in order to extend the in vivo lifetime, though it is not intended for BsAb preparation.

For example, as a method of stabilizing a peptide which specifically binds to a target protein, Patent Document 1 discloses expression and production of such a peptide, when it is composed only of natural amino acids, by gene recombination as a fusion product to be added to the terminal of the Fc region. Since the peptide is added to the terminal of the Fc region in this method, however, it is usually impossible to control a three-dimensional structure intrinsic to the peptide for securing high binding affinity and specificity to a target.

Patent Document 2 discloses a technology of inserting a peptide into the loop structure of an Fc region. The peptide thus inserted retains sufficient activity only when it is inserted in a certain one place (L139/T140), though many sites on the loop structure of the Fc region were tested as an insertion site of the peptide. According to the disclosure, when the peptide is inserted in another site, the fusion peptide however has a problem in expression in Escherichia coli or binding activity to a target molecule (Example 13, Tables 12 to 14). Further, conditions on what kind of physiologically active peptide can exhibit its activity again when inserted in such a limited loop structure of the Fc region are not shown clearly and it is difficult to select, from a considerable number of natural and non-natural (artificial) target-binding peptides, those suited for the creation of MsAb with a high success rate.

### Citation List

### Patent Documents

Patent Document 1: WO2001-083525
Patent Document 2: WO2006/036834

### Non-Patent Document

Non-Patent Document 1: Protein Eng Des Sel. 2010; 23(4): 289-297

### Summary

### Technical Problem

As described above, a technology of introducing a physiologically active peptide or the like into an antibody is known, but at present there is no efficient and versatile method capable of granting an additional binding specificity to an antibody.

The present invention is therefore completed in consideration of the above-described problem of the conventional technology. A purpose of it is to provide an efficient and versatile method for inserting a peptide sequence into an antibody and thereby granting an additional binding activity to the antibody. Another purpose of the present invention is to provide an engineered antibody obtained by the above method.

### Solution to Problem

As a result of intensive investigation, the present inventors have found that the above-described problems can be overcome by using, as a peptide sequence, the internal sequence of a cyclic peptide and completed the present invention.

The present invention is as follows:
(1) A method of granting a binding ability to a second target molecule to an antibody, comprising inserting an internal peptide sequence of a cyclic peptide into an Fc region of the antibody, wherein the cyclic peptide has the binding ability to the second target molecule different from a first target molecule to be recognized by the antibody.
(2) The method as described in (1), wherein the Fc region of the antibody is derived from human, mouse, rat, rabbit, horse, or dog.
(3) The method as described in (1) or (2), wherein a site into which the internal peptide sequence is inserted is a loop portion exposed on a molecular surface of the Fc region.
(4) The method as described in any of (1) to (3), wherein the cyclic peptide is a cyclic peptide obtained by a display type finding system.
(5) An engineered antibody having a binding ability to a first target molecule and a second target molecule, obtained by inserting an internal peptide sequence of a cyclic peptide into an Fc region of an antibody, wherein the cyclic peptide has the binding ability to the second target molecule different from the first target molecule to be recognized by the antibody.
(6) The engineered antibody as described in (5), wherein the engineered antibody simultaneously binds to the first and second target molecules.
(7) The engineered antibody as described in (5) or (6), wherein the first and second target molecules are respectively different cell surface molecules and the engineered antibody induces adhesion between heterologous cells via binding with two respectively different cell surface molecules.

### Advantageous Effects of Invention

The present invention makes it possible to freely grant an additional binding activity to an antibody by inserting, as a peptide sequence to be inserted in the antibody, an internal sequence of a cyclic peptide into the antibody.

### Brief Description of Drawings

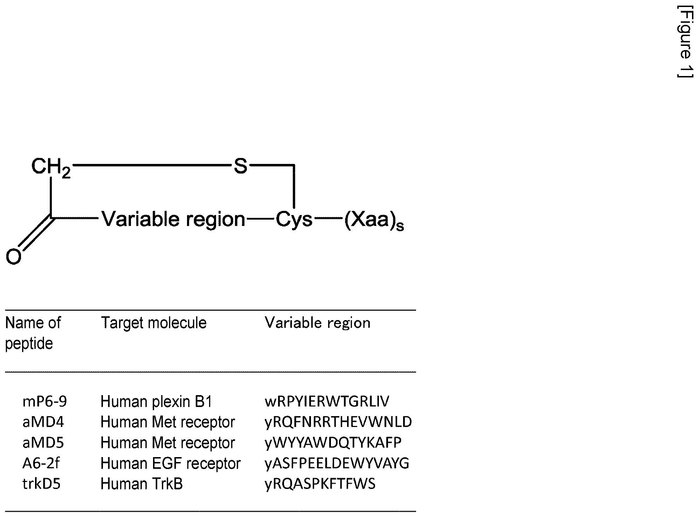

Fig. 1 shows the structure of cyclic peptides used in Examples. In the formula of the cyclic peptides, S represents a sulfur atom derived from the thiol group of Cys, Xaa represents an arbitrary amino acid, and s stands for an arbitrary integer of 0 or more. Variable region (variable region) shows an amino acid sequence constituting the internal structure of the cyclic peptide by one-letter code and the amino acid represented by a small letter (w, y, or the like) is an amino acid in D form (D-Trp, D-Tyr). SEQ ID NOS: 1 to 5 represent an amino acid sequence, among amino acid sequences represented as Variable region (variable region), except the amino acids in D form.

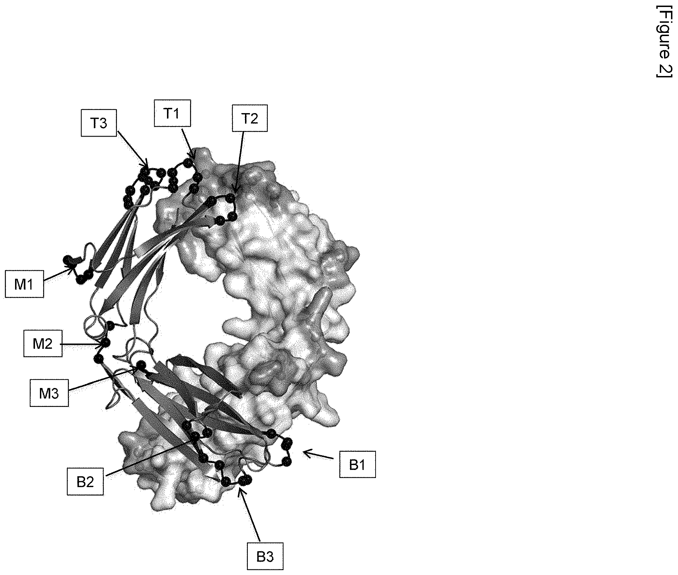

Fig. 2 shows the tertiary structure of an Fc region derived from human IgG (PDB 1D: 1FC1). One of the heavy chains constituting the Fc region is shown by a surface model (far side) and the other heavy chain is shown by a ribbon model (nearer side). A superficial loop structure capable of granting a novel property is indicated by 9 black Cα balls in the heavy chain shown by the ribbon model.

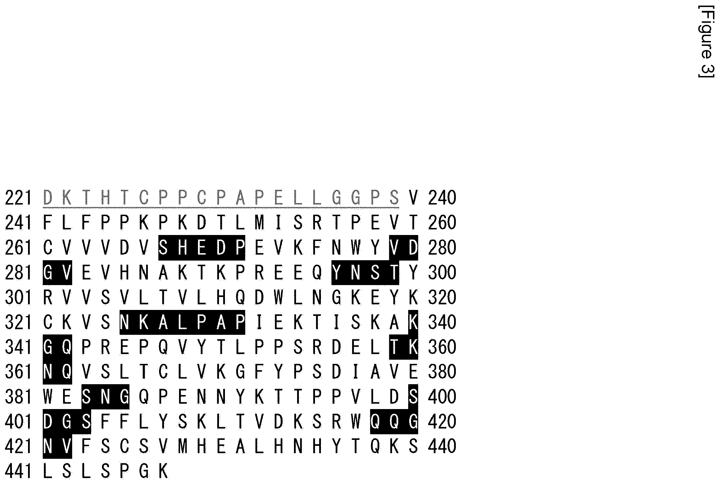

Fig. 3 shows the amino acid sequence of the hinge region and the Fc region of human IgG1 (SEQ ID NO: 6). The loop structure capable of granting a new specificity is indicated by a black background. The amino acid number is based on the numbering system of Chothia, et al. The underlined portion is a hinge region that connects the Fc region and the Fab region in the IgG molecule.

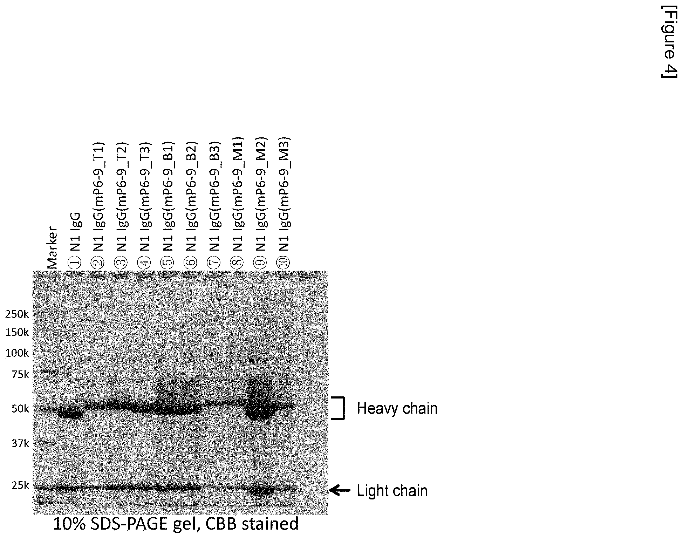

Fig. 4 shows SDS-PAGE of an anti-neuropilin-1 antibody (N1 IgG) expressed·secreted from Expi293F cells and engineered antibodies obtained by inserting a cyclic peptide (plexin B1-binding peptide mP6-9) into the antibody. For example, the term "mP6-9_T1" means that the cyclic peptide mP6-9 shown in Fig. 1 is inserted in the T1 site shown in Fig. 2.

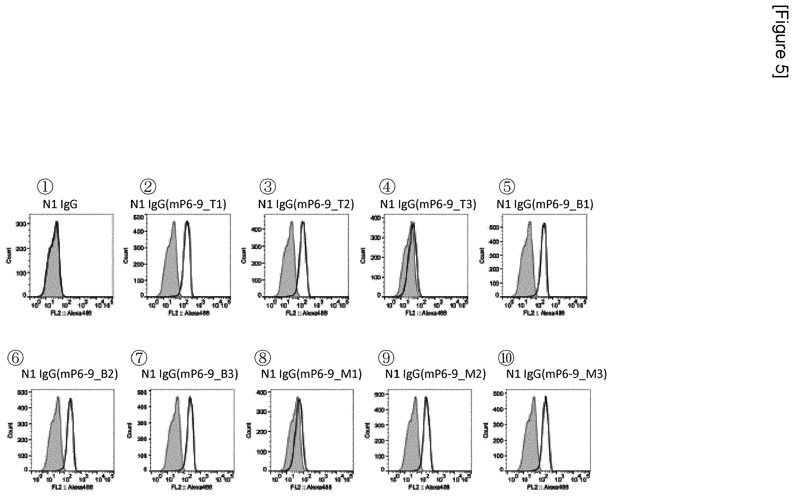

Fig. 5 shows the results of evaluating, by FACS, the binding of the anti-neuropilin-1 antibody (N1 IgG) and engineered antibodies having a cyclic peptide (plexin B1-binding peptide mP6-9) inserted therein to plexin B1 expressing cells. The joint histogram of a wild type N1 IgG before peptide insertion is indicated by a gray color, while the joint histogram of the mP6-9 insertion engineered antibody (insertion site is indicated on the upper part of each panel) is indicated by a thick solid line.

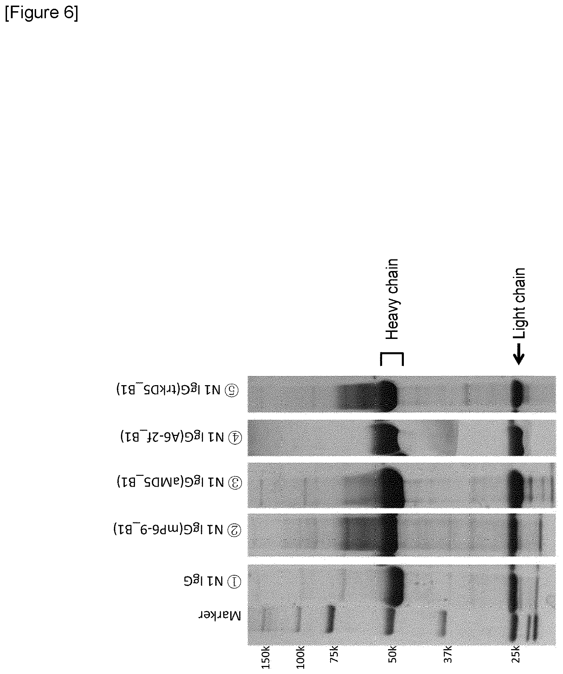

Fig. 6 shows SDS-PAGE of an anti-neuropilin-1 antibody (N1 IgG) expressed·secreted from Expi293F cells and engineered antibodies having various cyclic peptides inserted in the B1 site thereof.

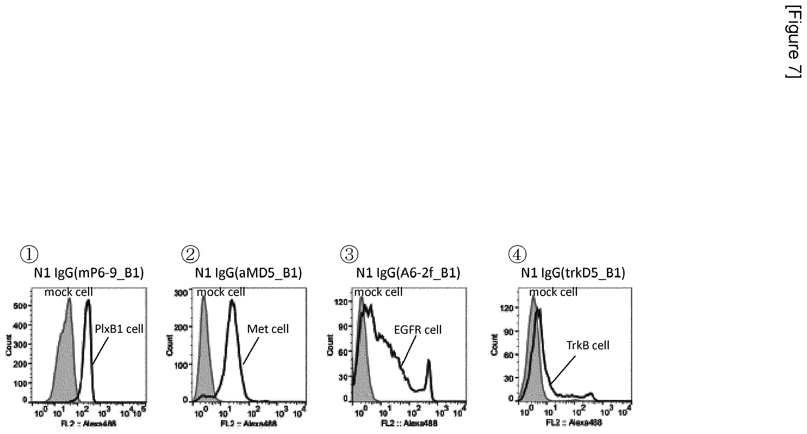

Fig. 7 shows the results of evaluating, by FACS, the binding of the anti-neuropilin-1 antibody (N1 IgG) and engineered antibodies having various cyclic peptides inserted in the B1 site thereof to second target molecules corresponding to the cyclic peptides. The joint histogram with control cells is indicated by a gray color, while the joint histogram with cells expressing the second target molecule (plexin B1 for mP6-9, Met for aMD5, EGFR for A6-2f, and TrkB for trkD5) is indicated by a thick solid line.

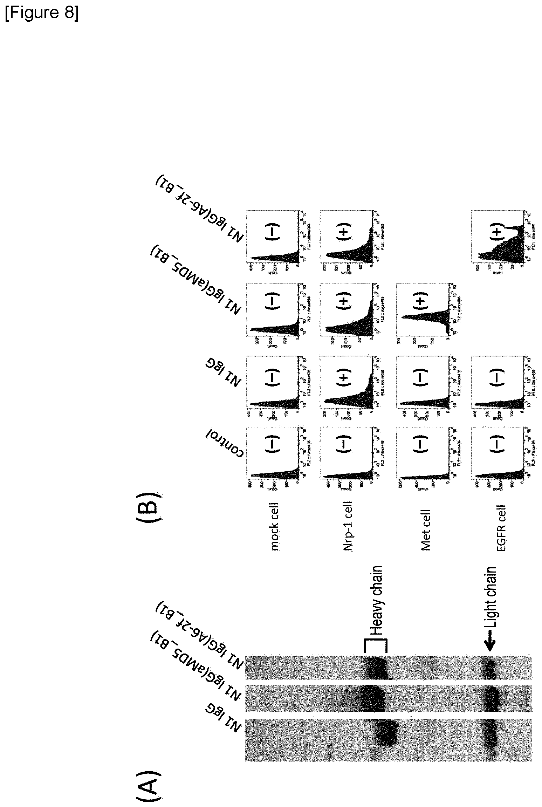

Fig. 8 shows SDS-PAGE (A) of an anti-neuropilin-1 antibody (N1 IgG) expressed·secreted from Expi293F cells and engineered antibodies having cyclic peptides inserted therein and joint histograms (B) of them with target molecules corresponding thereto according to FACS. When the signal obtained is 10 times or greater than the mean fluorescence intensity (mean fluorescence intensity) of a histogram (control) without an antibody, binding to a target molecule is taken as positive (+) and in the other case, it is taken as negative (-).

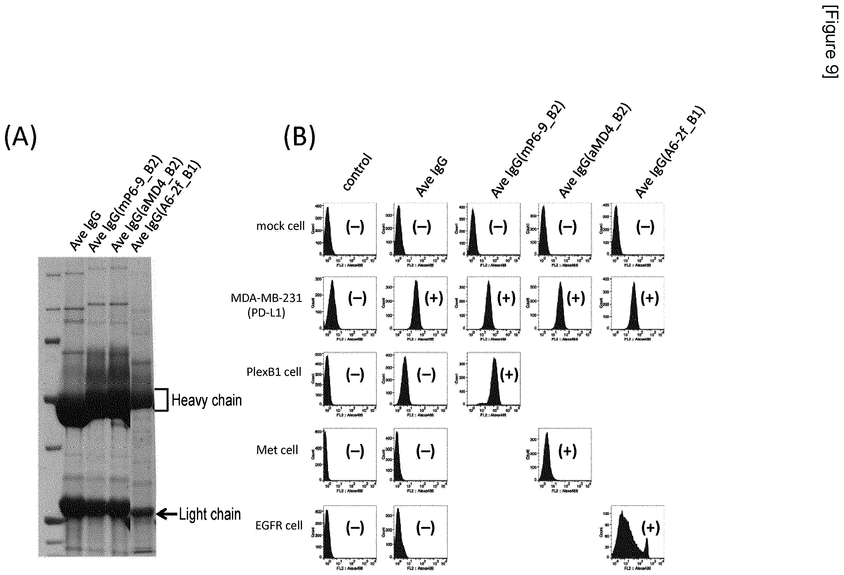

Fig. 9 shows SDS-PAGE (A) of an anti-PD-L1 antibody (Ave IgG) expressed·secreted from Expi293F cells and engineered antibodies having cyclic peptides inserted therein and joint histograms (B) of them with target molecules corresponding thereto according to FACS. When the signal obtained is 10 times or greater than the mean fluorescence intensity (mean fluorescence intensity) of a histogram (control) without an antibody, binding to a target molecule is taken as positive (+) and in the other case, it is taken as negative (-).

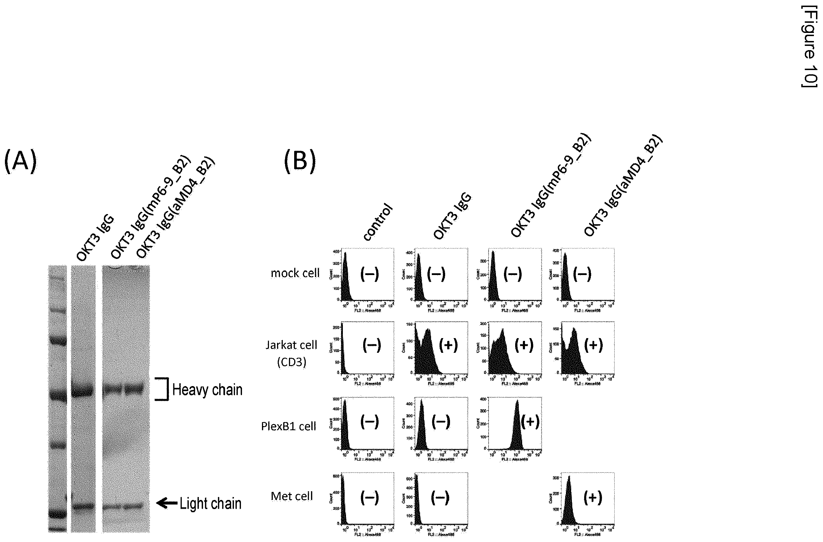

Fig. 10 shows SDS-PAGE (A) of an anti-CD3 antibody (OKT3 IgG) expressed·secreted from Expi293F cells and engineered antibodies having cyclic peptides inserted therein and joint histograms (B) of them with target molecules corresponding thereto according to FACS. When the signal obtained is 10 times or greater than the mean fluorescence intensity (mean fluorescence intensity) of a histogram (control) without an antibody, binding to a target molecule is taken as positive (+) and in the other case, it is taken as negative (-).

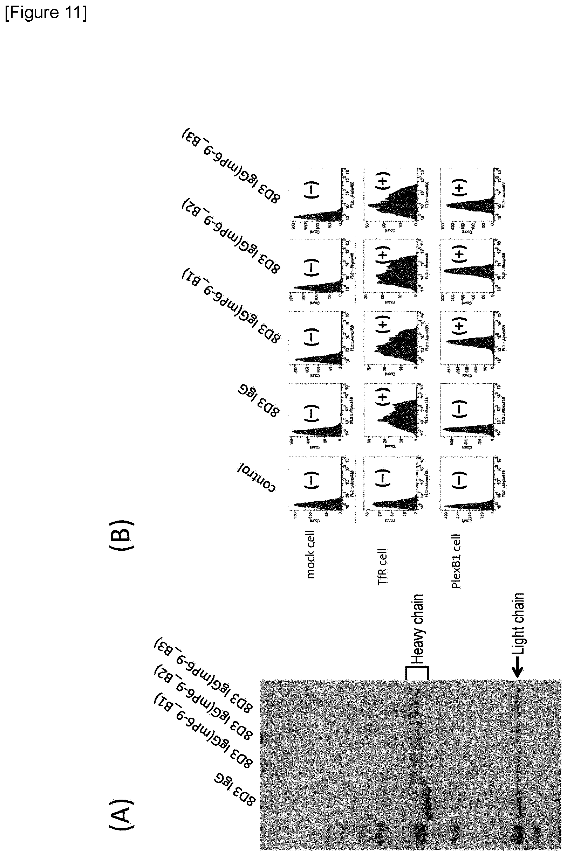

Fig. 11 shows SDS-PAGE (A) of an anti-transferrin receptor antibody (8D3 IgG) expressed·secreted from Expi293F cells and engineered antibodies having cyclic peptides inserted therein and joint histograms (B) of them with target molecules corresponding thereto. When the signal obtained is 10 times or greater than the mean fluorescence intensity (mean fluorescence intensity) of a histogram (control) without an antibody, binding to a target molecule is taken as positive (+) and in the other case, it is taken as negative (-).

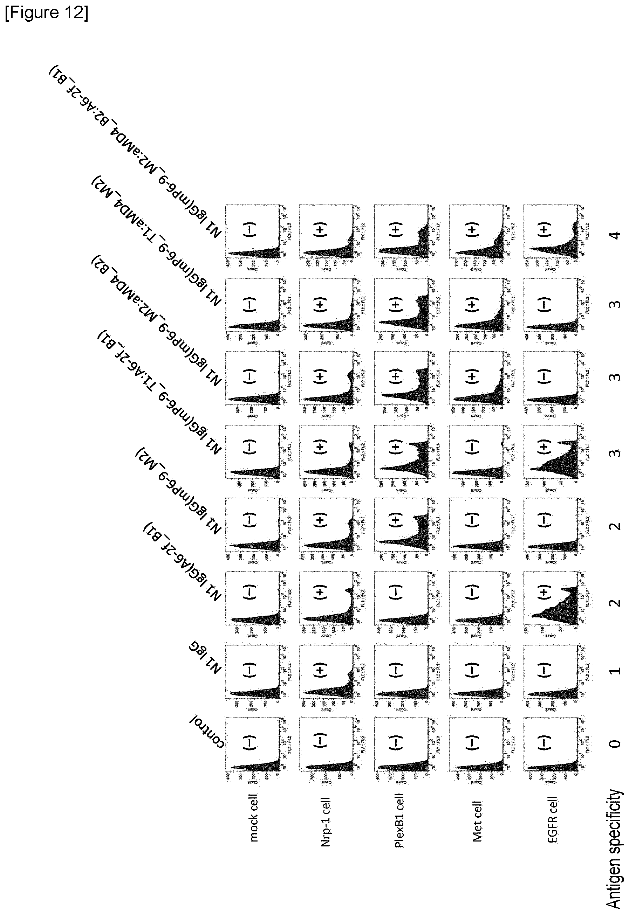

Fig. 12 shows joint histograms of an anti-neuropilin-1-antibody (N1 IgG) expressed·secreted from Expi293F cells and engineered antibodies having cyclic peptides inserted therein with target molecules corresponding thereto according to FACS. When a positive rate in binding to cells having a target molecule transiently expressed therein exceeds 10%, binding to the target molecule is taken as positive (+) and in the other case, it is taken as negative (?).

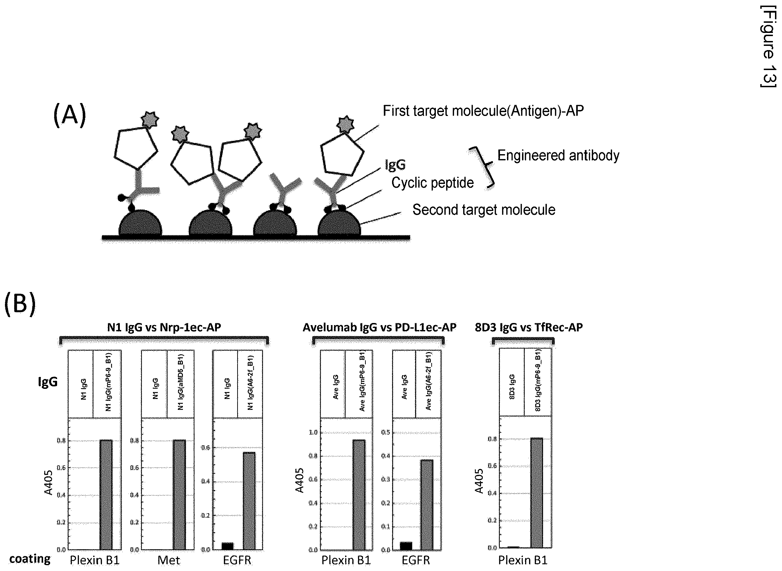

Fig. 13 shows the principle (A) of a sandwich measurement system showing that the engineered antibodies of the present invention each simultaneously bind to two kinds of target molecules and data (B) of them. In the graph, a used combination of an original antibody and an alkali phosphatase (AP) fusion protein of a first target molecule (antigen) is shown in the top of the graph; a second target molecule of the cyclic peptide coated on a microtiter plate is shown in the bottom of the graph, and the name of an antibody or Addbody added and color development (A405) of an AP substrate thus obtained is shown in in the middle.

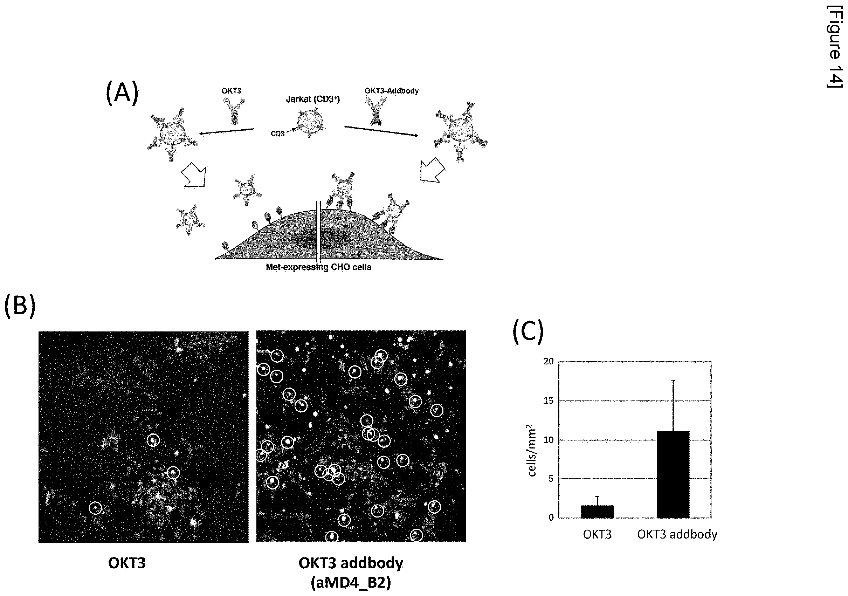

Fig. 14 shows a principle (A) of an adhesion inducing test of heterologous cells using a CD3/Met bispecific antibody (OKT3-Addbody) and data thereof (B and C). In the fluorescence microscope image shown in (B), a heterologous cell adhesion portion between Met expressing cells (labeled with a red fluorescent dye Dil) adhered onto a plate and CD3 expressing Jurkat cells (labeled with a green fluorescent dye DiO) is indicated by a white circle and measurement results (n=10) of it are indicated in (C).

### Description of Embodiments

The present invention will be described more specifically by a mode for carrying out the invention. The present invention is not limited to or by the following mode for carrying out the invention, but can be carried out after various modifications.

The present invention provides a method of inserting, into the Fc region of an antibody, an internal peptide sequence of a cyclic peptide having a binding ability (also called "binding specificity") to a second target molecule different from a first target molecule (can be understood also as an antigen) recognized by the antibody and thereby granting the binding ability to the second target molecule to the antibody.

According to the method of the present invention, a new binding specificity can be added to an arbitrary antibody that recognizes a specific target molecule and the engineered antibody thus obtained has not only a binding ability to the first target molecule of the original antibody but also a newly added binding ability to the second target molecule of the cyclic peptide.

In other words, in the method of the present invention, an additional binding activity can be freely granted to an antibody by inserting, as a peptide sequence to be inserted in the antibody, an internal sequence of a cyclic peptide into the antibody. In addition, in the method of the present invention, a cyclic peptide known to have a binding ability to a target molecule can be selected freely.

Although the cyclic peptide to be used in the present invention is not particularly limited insofar as it is a cyclic peptide known to have a binding ability to a target molecule, a natural cyclic peptide may be used or an non-natural cyclic peptide may be used.

Examples of the natural cyclic peptide include naturally occurring cyclic peptides and cyclic peptides composed of a natural amino acid, while examples of the non-natural cyclic peptide include cyclic peptides artificially synthesized using a non-natural amino acid.

When a natural cyclic peptide is presented on a protein, any bond for binding amino acid residues to each other is said to be a chemically crosslinked structure for forming an intramolecular cyclic structure so that any bond can be used as a bond for cleaving an intramolecular cyclic structure (closed-ring structure) to obtain a peptide sequence to inserted. The cyclic peptide is inserted in an antibody while conserving a region thought to be an active site of the natural cyclic peptide.

In the present invention, the cyclic peptide has a binding ability to a target molecule and this target molecule is different from a target molecule to which an antibody to be inserted has a binding ability originally.

In the present invention, the target molecule of the antibody is called "first target molecule" and the target molecule of the cyclic peptide is called "second target molecule". The first target molecule differs from the second target molecule.

Two or more cyclic peptides of the same kind can be used, but when two or more respectively different cyclic peptides are used, examples of a target molecule of a cyclic peptide other than the cyclic peptide having a binding ability to the second target molecule include a third target molecule, a fourth target molecule, and a target molecule other than the third and fourth target molecules. In the respectively different cyclic peptides, target molecules to which these cyclic peptides have a binding ability may be the same but are preferably different.

The term "cyclic peptide" means a peptide having, in the molecule thereof, at least a cyclic structure composed of four or more amino acid residues. The cyclic structure of the cyclic amino acids composed of four or more amino acid residues is a closed-ring structure formed in the molecule by bonding, directly or via a linker or the like, of two amino acid residues of a linear peptide separated from each other by two or more amino acids.

The term "two amino acid residues separated by two or more amino acids" means that the two amino acid residues have at least two amino acid residues therebetween. The two amino acid residues bond to each other with two or more amino acids therebetween.

The closed-ring structure in the cyclic structure is not particularly limited but it is formed by the covalent bonding of two amino acids.

Examples of the covalent bonding of two amino acids include disulfide bonding, peptide bonding, alkyl bonding, alkenyl bonding, ester bonding, thioester bonding, ether bonding, thioether bonding, phosphonate ether bonding, azo bonding, C-S-C bonding, C-N-C bonding, C=N-C bonding, amide bonding, lactam bridging, carbamoyl bonding, urea bonding, thiourea bonding, amine bonding, and thioamide bonding.

When two amino acids bond to each other at their main chain, a closed ring structure is formed by peptide bonding, but a covalent bond between two amino acids may be formed by bonding between the respective side chains of the two amino acids, bonding between the side chain and the main chain of them, or the like.

The cyclic structure is not limited to that formed by bonding between the N-terminal and C-terminal amino acids of a linear peptide, but it may be formed by bonding between a terminal amino acid and a non-terminal amino acid or bonding between non-terminal amino acids. When one of the amino acids bonded for the formation of a cyclic structure is a terminal amino acid and the other one is a non-terminal amino acid, the resulting cyclic peptide has a cyclic structure having, as a linear branched chain from the cyclic structure, a linear peptide attached thereto like a tail.

The amino acid that forms a cyclic structure may be a proteinogenic amino acid, an artificial amino acid mutant, or a derivative thereof. Examples include proteinogenic L-amino acids and chemically synthesized compounds having properties known in the art as characteristics of an amino acid.

The proteinogenic amino acids (proteinogenic amino acids) are, when represented by three-letter code known in the art, Arg, His, Lys, Asp, Glu, Ser, Thr, Asn, Gln, Cys, Gly, Pro, Ala, Ile, Leu, Met, Phe, Trp, Tyr, and Val.

The term "non-proteinogenic amino acids" (non-proteinogenic amino acids) means natural or non-natural amino acids other than proteinogenic amino acids.

Examples of the non-natural amino acids include α,α-disubstituted amino acids (such as α-methylalanine), N-alkyl-α-amino acids, D-amino acids, β-amino acids, and α-hydroxy acids, each having a main chain structure different from that of natural amino acids; amino acids (such as norleucine and homohistidine) having a sidechain structure different from that of natural amino acids; amino acids (such as "homo" amino acids, homophenylalanine, and homohistidine) having extra methylene in the side chain thereof; and amino acids (such as cysteic acid) obtained by substituting a carboxylic acid functional group in the side chain by a sulfonic acid group. Specific examples of the non-natural amino acids include amino acids described in WO2015/030014.

The number of amino acids constituting the cyclic structure is not particularly limited insofar as it is 4 or more. It may be, for example, 5 or more, 8 or more, or 10 or more and may be 30 or less, 25 or less, 20 or less, or 15 or less.

The number of amino acids constituting the cyclic structure is preferably 4 or more to 30 or less. Within a range of 4 or more to 30 or less, the number of amino acids constituting the cyclic structure may be set at 5 or more, 8 or more, or 10 or more and set at 25 or less, 20 or less, or 15 or less.

The number of amino acids constituting the cyclic structure may be set at 8 or more to 20 or less, 10 or more to 20 or less, or 10 or more to 15 or less.

The cyclic peptide to be used in the present invention is a cyclic peptide that can be produced using a known peptide synthesis technology.

Examples of a method of producing the cyclic peptide include a chemical synthesis method such as liquid-phase method, solid-phase method, or hybrid method using a liquid-phase method and a solid-phase method in combination; a gene recombination method, and a translation synthesis method in a cell-free translation system.

In the present invention, the cyclic peptide is preferably a cyclic peptide obtained by a display type finding system. A cyclic peptide having a binding ability to a target molecule can be selected by this display type finding system.

The cyclic peptide may be any of a peptide produced by a conventional mRNA display method, a peptide produced by an mRNA display method such as TRAP or RaPID or a peptide produced by a phage display method. The cyclic peptide may be a peptide produced by a modified method thereof.

The cyclic peptide contains, for example, a thioether bond or a disulfide bond as the chemically crosslinked structure for forming an intramolecular cyclic structure.

Typically in the cyclic peptide selected by the mRNA display method such as RaPID or TRAP or the phage display method, a structure other than the chemically crosslinked structure for forming an intramolecular cyclic structure such as thioether bond or disulfide bond tends to be an active spot having physiological activity.

Then, by replacing the thioether bond or disulfide bond of the cyclic peptide with a bond with a protein having a loop structure, high specificity and affinity of a cyclic peptide ordinarily available by the mRNA display method such as RaPID or TRAP or the phage display method can be bestowed to an intended loop structure of an intended protein. Although not particularly limited, by fusing a cyclic peptide having a thioether bond or disulfide bond as an intramolecular cyclic structure, a cyclic peptide can be fused with versatility.

The RaPID method can produce, for example, a cyclic peptide in which an amino acid having Functional group 1 and an amino acid having Functional group 2 corresponding thereto, each shown below in Table 1, have been cyclized.

Either Functional group 1 or 2 may be placed on the N-terminal side; they may be placed at the N-terminal and C-terminal, respectively; one of them may be a terminal amino acid and the other one may be a non-terminal amino acid; or both may be a non-terminal amino acid.

**Table 1:**

| | Functional group 1 | Functional group 2 |
|---|---|---|
| (A) | 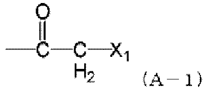 | HS- (A-2) |
| (B) | -C≡C-H (B-1) | N₃- (B-2) |
| (C) | -Ar-CH₂NH₂ (C - 1) | 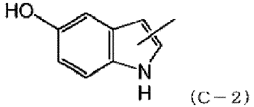 |
| (D) | -C≡C-CH₂-X₁ (D - 1) | HS- (D-2) |
| (E) | -Ar-CH₂-X₁ (E - 1) | HS- (E-2) |

In the above formulas, X₁ represents a leaving group and examples of the leaving group include a halogen atom such as CI, Br, or I and Ar represents a substituted or unsubstituted aromatic ring.

In the phage display method, a cyclic peptide in which two Cys bond to each other to form a cycle can be obtained so that a cyclic peptide having a disulfide bond between -SH as Functional group 1 and HS- as Functional group 2 can be obtained.

As the amino acid having Functional group (A-1), for example, a chloroacetylated amino acid can be used. Examples of the chloroacetylated amino acid include N-chloroacetyl-L-alanine, N-chloroacetyl-L-phenylalanine, N-chloroacetyl-L-tyrosine, N-chloroacetyl-L-tryptophan, N-3-(2-chloroacetamido)benzoyl-L-phenylalanine, N-3-(2-chloroacetamido)benzoyl-L-tyrosine, N-3-(2-chloroacetamido)benzoyl-L-tryptophan, β-N-chloroacetyl-L-diaminopropanoic acid, γ-N-chloroacetyl-L-diaminobutyric acid, σ-N-chloroacetyl-L-ornithine, and ε-N-chloroacetyl-L-lysine, and D-amino acid derivatives corresponding thereto.

As the amino acid having Functional group (A-1), N-chloroacetyl-L-tryptophan and N-chloroacetyl-L-tyrosine are preferably used, with D-form of it being more preferred.

In the present specification, an amino acid is sometimes described clearly as L-form but it may be either L-form or D-form. It may also be a mixture of L-form and D-form at any ratio. Even when an amino acid is described without clearly showing that it is in L-form or D-form, it may be either L-form or D-form, or a mixture of L-form and D-form at any ratio.

Examples of the amino acid having Functional group (A-2) include cysteine, homocysteine, mercaptonorvaline, mercaptonorleucine, 2-amino-7-mercaptoheptanoic acid, and 2-amino-8- mercaptooctanoic acid.

As the amino acid having Functional group (A-2), cysteine is preferably used.

Examples of the cyclization method using the amino acid having Functional group (A-1) and the amino acid having Functional group (A-2) include those described in Kawakami, T. et al., Nature Chemical Biology 5, 888-890 (2009);Yamagishi, Y. et al., ChemBioChem 10, 1469-1472 (2009); Sako, Y. et al., Journal of American Chemical Society 130, 7932-7934 (2008); Goto, Y. et al., ACS Chemical Biology 3, 120-129 (2008); Kawakami T. et al, Chemistry & Biology 15, 32-42 (2008); and WO2008/117833.

Examples of the amino acid having Functional group (B-1) include propargylglycine, homopropargylglycine, 2-amino-6-heptynoic acid, 2-amino-7-octynoic acid, and 2-amino-8-nonynoic acid.

Alternatively, a 4-pentynoylated or 5-hexynoylated amino acid may be used.

Examples of the 4-pentynoylated amino acid include N-(4-pentenoyl)-L-alanine, N-(4-pentenoyl)-L-phenylalanine, N-(4-pentenoyl)-L-tyrosine, N-(4-pentenoyl)-L-tryptophan, N-3-(4-pentynoylamido)benzoyl-L-phenylalanine, N-3-(4-pentynoylamido)benzoyl-L-tyrosine, N-3-(4-pentynoylamido)benzoyl-L-tryptophan, β-N-(4-pentenoyl)-L-diaminopropanoic acid, γ-N-(4-pentenoyl)-L-diaminobutyric acid, σ-N-(4-pentenoyl)-L-ornithine, and ε-N-(4-pentenoyl)-L-lysine, and D-amino acid derivatives corresponding thereto.

Examples of the 5-hexynoylated amino acid include amino acids obtained by substituting the 4-pentynoyl group of the compounds exemplified as the 4-pentynoylated amino acid by a 5-hexynoyl group.

Examples of the amino acid having Functional group (B-2) include azidoalanine, 2-amino-4-azidobutanoic acid, azidoptonorvaline, azidonorleucine, 2-amino-7-azidoheptanoic acid, and 2-amino-8- azidooctanoic acid.

Alternatively, an azidoacetylated or 3-azidopentanoylated amino acid can also be used.

Examples of the azidoacetylated amino acid include N-azidoacetyl-L-alanine, N-azidoacetyl-L-phenylalanine, N-azidoacetyl-L-tyrosine, N-azidoacetyl-L-tryptophan, N-3-(4-pentynoylamido)benzoyl-L-phenylalanine, N-3-(4-pentynoylamido)benzoyl-L-tyrosine, N-3-(4-pentynoylamido)benzoyl-L-tryptophan, β-N-azidoacetyl-L-diaminopropanoic acid, γ-N-azidoacetyl-L-diaminobutyric acid, σ-N-azidoacetyl-L-ornithine, and ε-N-azidoacetyl-L-lysine, and D-amino acid derivatives corresponding thereto.

Examples of the 3-azidopentanoylated amino acid include amino acids obtained by substituting the azidoacetyl group of the compounds exemplified as the azidoacetylated amino acid by a 3-azidopentanoyl group.

Examples of the cyclization method using the amino acid having Functional group (B-1) and the amino acid having Functional group (B-2) include the methods described in Sako, Y. et al., Journal of American Chemical Society 130, 7932-7934 (2008) and WO2008/117833.

Examples of the amino acid having Functional group (C-1) include N-(4-aminomethyl-benzoyl)-phenylalanine (AMBF) and 3-aminomethyltyrosine.

Examples of the amino acid having Functional group (C-2) include 5-hydroxytryptophan (WOH).

Examples of the cyclization method using the amino acid having Functional group (C-1) and the amino acid having Functional group (C-2) include the methods described in Yamagishi, Y. et al., ChemBioChem 10, 1469-1472 (2009) and WO2008/117833.

Examples of the amino acid having Functional group (D-1) include 2-amino-6-chloro-hexynoic acid, 2-amino-7-chloro-heptynoic acid, and 2-amino-8-chloro-octynoic acid.

Examples of the amino acid having Functional group (D-2) include cysteine, homocysteine, mercaptonorvaline, mercaptonorleucine, 2-amino-7-mercaptoheptanoic acid, and 2-amino-8-mercaptooctanoic acid.

Examples of the cyclization method using the amino acid having Functional group (D-1) and the amino acid having Functional group (D-2) include the method described in WO2012/074129.

Examples of Amino acid (E-1) include N-3-chloromethylbenzoyl-L-phenylalanine, N-3-chloromethylbenzoyl-L-tyrosine, and N-3-chloromethylbenzoyl-L-tryptophan and D-amino acid derivatives corresponding thereto.

Examples of Amino acid (E-2) include cysteine, homocysteine, mercaptonorvaline, mercaptonorleucine, 2-amino-7-mercaptoheptanoic acid, and 2-amino-8- mercaptooctanoic acid.

The cyclization method using the amino acid having Functional group (E-1) and the amino acid having Functional group (E-2) can be carried out referring to, for example, the cyclization method using (A-1) and (A-2) or the cyclization method using (D-1) and (D-2).

The ring-forming amino acid is preferably a combination of the amino acid having Functional group (A-1) with the amino acid having Functional group (A-2), more preferably a combination of N-acetyltryptophan obtained by substituting H by a leaving group with cysteine, still more preferably combination of an N-haloacetyl-D-tyrosine or an N-haloroacetyl-D-tryptophan, preferably, N-chloroacetyl-D-tyrosine or N-chloroacetyl-D-tryptophan with cysteine (Cys).

In the present invention, a cyclic peptide obtained by a display type finding system can be used as the cyclic peptide having a binding ability to a predetermined target molecule.

The cyclic peptide is preferably a cyclic peptide produced by the mRNA display method such as RaPID or TRAP or the phage display method.

It is preferably a cyclic peptide which can be preferably produced by the mRNA display method such as RaPID or TRAP or the phage display method and is formed from Functional group 1 and Functional group 2.

In the present invention, the term "cyclic peptide has a binding ability to a target molecule" preferably means that it has an affinity value (K_{D} value of approximately 1 µM or less) on an equal level to a value that an antibody usually exhibits to its target molecule, though it is not particularly limited insofar as the cyclic peptide can bind to its target molecule.

The cyclic peptide may function as an inhibitor or activator as well as having a binding ability to a target molecule, or it may have an agonist activity or antagonist activity.

The engineered antibody of the present invention preferably has, as a binding activity to a second target molecule corresponding to the cyclic peptide, 10% or more of the binding activity of an original cyclic peptide to the second target molecule. It may have 20% or more, 30% or more, 40% or more, 50% or more, 60% or more, 70% or more, 80% or more, 90% or more, 95% or more, 96% or more, 97% or more, 98% or more, 99% or more, 100% or more of the binding activity.

Although the target molecule is not particularly limited even if it is the first target molecule, the second target molecule, or another target molecule, examples include molecules on a cell surface such as cell adhesion receptors, cytokine receptors, growth factor receptors, immunoreceptors, signal molecule receptors, G protein conjugated receptors, transferrin receptors, transporters, and channel molecules; and soluble molecules such as growth factors, cytokines, signal molecules, transferrin, blood coagulation factors, and extracellular matrix proteins.

The cyclic peptide to be used in the present invention will hereinafter be described with a cyclic peptide produced by the RaPID method as an example.

In the present invention, the cyclic peptide produced by the RaPID method is not particularly limited but a cyclic peptide represented by the following formula can be given as an example.

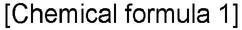

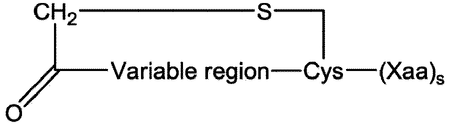

The cyclic peptide represented by the above formula is an example and it has a structure having, as Functional group 1 and Functional group 2, those listed in (A) in Table 1.

In the formula, S represents a sulfur atom derived from the thiol group of Cys, Xaa represents an arbitrary amino acid, and s stands for an arbitrary integer of 0 or more. The term "Variable region" (variable region) means an amino acid sequence, except Cys, constituting a cyclic amino acid. The N-terminal amino acid of Variable region (variable region) is preferably an amino acid having Functional group (A-1).

The amino acid sequence of Variable region (variable region) is a partial amino acid sequence of the cyclic peptide produced by the RaPID method and it may be any amino acid sequence insofar as it forms the cyclic peptide.

When the peptide represented by the above formula is used as the cyclic peptide (Cyclic Peptide is sometimes called "CP"), the N-terminal amino acid residue of Variable region is called "CP_{N}" and Cys is called "CPc".

When CP_{N} is, for example, an N-haloroacetyl-D-tryptophan which is a non-proteinogenic amino acid, it is preferably substituted with L-tryptophan which is a proteinogenic amino acid, while when it is, for example, N-chloroacetyl-D-tyrosine which is a non-proteinogenic amino acid, it is preferably substituted with L-tyrosine which is a proteinogenic amino acid. Then, fusion of the cyclic peptide preferably follows. Alternatively, CP_{N} may be substituted with Cys which is a proteinogenic amino acid. When CPc is, for example, Cys, the cyclic peptide is preferably fused while deleting CPc. When CPc is Cys and also Cys which is CPc is fused, CP_{N} is preferably substituted with Cys, followed by fusion. When the cyclic peptide represented by the above formula is inserted, a D-amino acid is substituted by an L-amino acid if desired but the amino acid sequence of Variable region (variable region) is preferably inserted as a partial amino acid sequence of the amino acid sequence forming the cyclic structure of the cyclic peptide.

In the present invention, it is understood that even when a portion of the amino acid sequence of Variable region (variable region) - for example, in the case where CP_{N} is a non-proteinogenic amino acid - is substituted by a proteinogenic amino acid, an antibody has, inserted therein, the amino acid sequence of Variable region (variable region). Variable region (variable region) and Cys as CPc may both be fused.

When a cyclic peptide preferably produced by the mRNA display method such as RaPID or TRAP or the phage display method is inserted into an antibody, it is also preferred to carry out engineering as described below.
(1) Amino acid residues involved in the chemically crosslinked structure for forming an intramolecular cyclic structure in the mRNA display method such as RaPID or TRAP or the phage display method, for example, amino acid residues having the functional group described in Table 1, more specifically, amino acids described as (A-1) to (E-2) may each be substituted or removed and the internal amino acid sequence of the cyclic peptide may be inserted into an antibody.
   More specifically, in the cyclic peptide produced by the RaPID method, the amino acid residue having Functional group 1 is substituted by a proteinogenic amino acid and the amino acid residue having Functional group 2 is deleted, followed by insertion into an antibody. As the amino acid residue having Functional group 1, a non-proteinogenic amino acid such as D-amino acid is sometimes used.
   In the cyclic peptide produced by the phage display method, a Cys residue forming an S-S bond, a chemically crosslinked structure, may be removed, followed by insertion into an antibody.
   More specifically, in the RaPID method, when the structure (A-1) is used for Functional group 1, it is the common practice to use CIAc-D-Trp or CIAc-D-Tyr as an amino acid having the structure (A-1), but insertion into an antibody is preferably performed after the amino acid residue is substituted by L-Trp or L-Tyr. Alternatively, CIAc-D-Trp or CIAc-D-Tyr may be deleted.
   As the amino acid having the structure (A-2), Cys is often used, but insertion into an antibody may be performed after the Cys residue is deleted.
(2) In (1), an amino acid sequence not having a cyclic peptide but composed of an amino acid residue such as Ser, Gly and Cys is used as a linker sequence between the cyclic peptide and an antibody and it may be inserted between the amino acid residue derived from the cyclic peptide and the amino acid residue derived from the antibody.
(3) In an antibody engineered by changing an amino acid residue involved in the chemically crosslinked structure for forming an intramolecular cyclic structure to L-Cys and inserting the cyclic peptide into the antibody, a crosslinked structure may be formed by disulfide bonding and at the same time, an amino acid sequence composed of an amino acid residue such as Ser and Gly may be used as a linker sequence for bonding between the cyclic peptide and the antibody and inserted between the amino acid residue derived from the cyclic peptide and the amino acid residue derived from the antibody.
   The number of the amino acid residues constituting the linker sequence may be one or more and the number of the amino acid residues is not particularly limited.

The internal peptide sequence of the cyclic peptide is not particularly limited insofar as it is a peptide sequence constituting the cyclic peptide and contains a region having a binding ability to a target molecule.

The following description will be made supposing that the primary sequence of the cyclic peptide is CP_{N}-(Xaa1)ₘ-CP_{C}.

In the cyclic peptide, CP_{N} and CPc, of the amino acid residues represented by CP_{N}-(Xaa1)ₘ-CP_{C}, preferably bind to each other covalently and form a cyclic structure.

It is to be noted that in the primary sequence, Xaa1 is an arbitrary amino acid residue and m stands for an arbitrary integer of 2 or more insofar as a cyclic peptide can be formed.

The cyclic peptide may have a chain-like branched chain not only from the intramolecular cyclic structure but also from the cyclic structure.

The internal peptide sequence of the cyclic peptide may be the primary sequence CP_{N}-(Xaa1)ₘ-CP_{C} itself or some of the amino acid residues of CP_{N}-(Xaa1)ₘ-CP_{C} may be substituted or deleted. CP_{N}-(Xaa1)ₘ can be given as an example of partially deleted amino acids

The internal peptide sequence of the cyclic peptide is only required to contain, among (m+2) pieces of the amino acid residues represented by CP_{N}-(Xaa1)ₘ-CP_{C}, at least some of the (m+2) pieces of the amino acid residues and CP_{N}-(Xaa1)ₘ1, (Xaa1)ₘ1-CP_{C}, or (Xaa1)ₘ1 can be used as the internal peptide sequence of the cyclic peptide.

In the above description, m1 is an integer of m or less.

When the internal peptide sequence of the cyclic peptide is inserted in an antibody, it may be inserted in the antibody via a linker sequence if desired.

When the cyclic peptide contains, in the internal peptide sequence thereof, a non-proteinogenic amino acid, the non-proteinogenic amino acid is preferably substituted by a proteinogenic amino acid. When CP_{N} and/or CPc is Cys, CP_{N} and/or CPc may be deleted and when CPc is Cys, the cyclic peptide may be inserted after substitution of CP_{N} by Cys.

When the internal peptide sequence of the cyclic peptide is inserted in an antibody, amino acids derived from the cyclic peptide may be bound at CP_{N}, may be bound at CP_{N} via a linker sequence, may be bound at the (arbitrary number)-th Xaa1 counted from CP_{N}, or may be bound at the (arbitrary number)-th Xaa1 counted from CP_{N} via a linker sequence. Particularly when CP_{N} is a non-proteinogenic amino acid, CP_{N} may be substituted with a proteinogenic amino acid, followed by binding via a linker sequence if desired.

When the internal peptide sequence of the cyclic peptide is inserted in an antibody, amino acids derived from the cyclic peptide may be bound at CPc, may be bound at CPc via a linker sequence, may be bound at the (arbitrary number)-th Xaa1 counted from CPc, or may be bound at the (arbitrary number)-th Xaa1 counted from CPc via a linker sequence. Above all, CPc may be deleted, followed by binding via a linker sequence if desired at the first Xaa1 counted from CPc, in other words, at Xaa1 to be bound to CP_{C}.

In the present invention, the antibody to be inserted with the internal peptide sequence of the cyclic peptide is not particularly limited.

The antibody is also called immunoglobulin and is classified into five kinds, that is, IgG, IgM, IgA, IgD, and IgE.

In the present invention, the antibody to be inserted with the cyclic peptide may be any of IgG, IgM, IgA, IgD, and IgE, with IgG being preferred.

As IgG, many subclasses are known. As the subclasses of IgG, for example, four kinds of subclasses, that is, IgG1, IgG2, IgG3, and IgG4 are known in human, four kinds, that is, IgG1, IgG2a, IgG2b, and IgG3 are known in mouse, and four kinds, that is, IgG1, IgG2a, IgG2b, and IgG2c are known in rat. Any of them may be used.

Since an antibody essentially binds specifically to, as a target molecule, only one antigen molecule, an antigen recognized by the antibody can be said as the first target molecule in the present invention.

Antibodies each have two heavy chains and two light chains each having a variable region which can be regarded as an antigen recognizing site and a constant region which is a region other than the variable region.

Digestion of an antibody with protease papain cleaves between disulfide bonds (hinge site) that connect the H chain-H chain and the antibody is separated into three fragments. The two N-terminal fragments are called a Fab region and the C-terminal fragment is called a Fc region.

In antibodies of the same subclass, their Fc regions usually have the same structure.

The antibody to be used in the present invention has at least one Fab region composed of N-terminal-side two fragments and an Fc region. In the antibody containing two Fab regions, these two Fab regions may be the same or different. When the Fab regions are different, target molecules recognized by the antibody may be the same or different.

When the Fab regions are different and the target molecules recognized by the antibody are different, the antibody retains a binding ability to, as the first target molecule, two target molecules (first target molecule and a target molecule different therefrom). The binding ability to a second target molecule which the cyclic peptide has differs from the binding ability to the first target molecule. Although it may be a binding ability to the target molecule different from the first target molecule, it is preferably different from the binding ability to the first target molecule serving as the target molecule of the antibody and to the target molecule different therefrom. When the cyclic peptide has a binding ability to a target molecule other than the second target molecule, the third, fourth or another target molecule may also be different from the target molecule recognized by the antibody such as the first target molecule.

In the present invention, the internal peptide sequence of the cyclic peptide is inserted in the Fc region of the antibody.

An insertion site in the Fc region of the antibody for inserting the internal peptide sequence of the cyclic peptide is not particularly limited in the present invention, but the insertion site is preferably a loop portion exposed on the molecular surface in the Fc region in order to grant a binding ability to the second target molecule of the cyclic peptide to the antibody while retaining the binding ability of the antibody to the first target molecule.

The loop portion is preferably a polypeptide chain region, in the Fc region, that connects between two β strands, has a folded structure, and has a structure exposed from the molecular surface of the antibody.

In the present invention, when the cyclic peptide is inserted in the antibody, the bond between an amino acid residue derived from the cyclic peptide and an amino acid residue derived from the antibody is preferably a covalent bond.

A peptide chain serving as a linker may be inserted between the amino acid residue derived from the cyclic peptide and the amino acid residue derived from the antibody.

The loop portion into which the internal peptide sequence of the cyclic peptide is inserted is not particularly limited in the loop structure in the Fc region of the antibody and any site in the loop structure may be selected with versatility.

The term "any site in the loop structure" means that two amino acid residues for inserting the cyclic peptide are each present at a site selected from the loop structure.

Although the insertion site is not particularly limited, it will be described referring to Fig. 2 and Fig. 3.

Fig. 2 shows the tertiary structure of a human IgG-derived Fc region. One of heavy chains constituting Fc is indicated as a surface model (far side) and the other one is indicated by a ribbon model (nearer side).

The loop portion preferably selected as the insertion site of the cyclic peptide in the present invention is shown in Fig. 2 and portions indicated by T1 to T3, M1 to M3, and B1 to B3 are corresponding portions.

Fig. 3 shows the amino acid sequence of the Fc region derived from human IgG1 and the loop portion selected preferably as the insertion site of the cyclic peptide is indicated by a black background.

Preferable examples of the loop portion selected preferably include:
T1 (SHEDP, SEQ ID NO: 8): positions 267 to 271,
T2 (YNST, SEQ ID NO: 9) : positions 296 to 299,
T3 (NKALPAP, SEQ ID NO: 10) : positions 325 to 331,
M1 (VDGV, SEQ ID NO: 11) : positions 279 to 282,
M2 (KGQ, SEQ ID NO: 12) : positions 340 to 342,
M3 (SDGS, SEQ ID NO: 13) : positions 400 to 403,
B1 (TKNQ, SEQ ID NO: 14) : positions 359 to 362,
B2 (SNG, SEQ ID NO: 15) : positions 383 to 385, and
B3 (QQGNV, SEQ ID NO: 16) : positions 418 to 422, each of the human IgG1-derived Fc region represented by SEQ ID NO: 7.

With T1 as an example, the positions 267 to 271 correspond to positions 47 to 51 in SEQ ID NO: 6 and positions 28 to 32 in SEQ ID NO: 7. This means that positions indicated as T2 to T3, M1 to M3, and B1 to B3 correspond to amino acids at positions obtained by subtracting 220 in SEQ ID NO: 6 and amino acids at positions obtained by subtracting 239 in SEQ ID NO: 7.

The insertion site of the cyclic peptide may be a site selected from the loop structure indicated by the position of an amino acid residue. With T1 as an example, positions 267 and 268, positions 268 and 269, positions 269 and 270, positions 270 and 271, positions 267 and 269, positions 268 and 270, positions 269 and 271, positions 267 and 270, positions 268 and 271, and positions 267 and 271 can be selected as the site selected from the loop structure and thus-selected amino acid residue positions may be insertion sites of the internal amino acid sequence of the cyclic peptide into the antibody.

In an antibody other than human IgG1, a portion corresponding to the portion indicated by T1 to T3, M1 to M3, and B1 to B3 in alignment with the amino acid sequence represented by SEQ ID NO: 6 or SEQ ID NO: 7 can be selected as the insertion site of the cyclic peptide.

More preferably, in the loop portion of the human IgG1-derived Fc region represented by SEQ ID NO: 7, following positions marked with * between amino acid residues:
T1 (SH*EDP): positions 267 to 271,
T2 (Y*NST): positions 296 to 299,
T3 (NKALP*AP): positions 325 to 331,
M1 (VD*GV): positions 279 to 282,
M2 (K*GQ): positions 340 to 342,
M3 (SD*GS): positions 400 to 403,
B1 (TK*NQ): positions 359 to 362,
B2 (S*NG): positions 383∼385, and
B3 (QQ*GNV): positions 418 to 422
are more preferable sites for insertion of the cyclic peptide.

By inserting the internal peptide sequence of the cyclic peptide, amino acid residues derived from the cyclic peptide or two amino acid residues of the loop portion of the antibody to be bound to the amino acid residue of a linker may be, in the loop structure, contiguous to each other or two amino acid residues in a relation that two amino acid residues are discontinuous and have therebetween 1 to 15 amino acid residues in the Fc region of the antibody.

Although not particularly limited in the present invention, by inserting different internal peptide sequences derived from the cyclic peptide into the loop portion selected preferably as an insertion site, the resulting antibody can have, in addition to the binding ability intrinsic thereto, binding specificities to second, third, and fourth target molecules or target molecules other than the first to fourth ones to which the cyclic peptide has a binding activity.

As the antibody for obtaining a multispecific antibody, any type of an existing antibody may be used as an original one and as the cyclic peptide, any cyclic peptide may be used insofar as its binding ability to a second target molecule is known.

Since an antibody to be used, an internal peptide sequence of a cyclic peptide to be inserted, and an insertion site thereof can be used in any combination, an engineered antibody having desired multispecificity can be created very conveniently if antibodies and cyclic peptides that bind to various antigens are prepared in advance.

The multispecific antibody available by the present invention can be used as various drugs such as those having efficacy equal to antibody drugs using two kinds of single IgG antibodies in combination and those recognizing target molecules on different cells simultaneously and inducing crosslinking between heterologous cells.

The antibody to be used in the present invention is not particularly limited and examples include mouse antibodies, rat antibodies, rabbit antibodies, horse antibodies, dog antibodies, chimeric antibodies, humanized antibodies, and fully human antibodies.

The Fc region of the antibody into which the cyclic peptide is inserted is preferably derived from any of humans, mice, rats, rabbits, horses, and dogs.

The insertion of the internal peptide sequence of the cyclic peptide into the Fc region of the antibody in the present invention will next be described more specifically.

Supposing that, in two amino acid residues which are present in the Fc region of the antibody and bind to the internal peptide sequence of the cyclic peptide, the amino acid residue situated on the N terminal side is called AB_{N} and the amino acid residue on the C terminal side is called ABc, the internal peptide sequence of the cyclic peptide to be inserted may bind to AB_{N} or bind to AB_{N} via a linker sequence.

The internal peptide sequence of the cyclic peptide to be inserted may bind to ABc or bind to ABc via a linker sequence.

AB_{N} and ABc may be continuous or discontinuous as a loop structure in the Fc region.

When they are discontinuous, AB_{N} and ABc preferably contain 1 to 5 amino acid residues therebetween.

In the present invention, a cyclic peptide is inserted into the Fc region of an antibody to grant a binding ability to a target molecule which the cyclic peptide inserted in the antibody to the antibody has and this insertion of the cyclic peptide into the Fc region of the antibody can be achieved by typical gene engineering technology.

The present invention provides, as well as a method of inserting a cyclic peptide into the Fc region of an antibody, an engineered antibody obtained by inserting a cyclic peptide into the Fc region of an antibody and thereby having the internal peptide sequence of the cyclic peptide inserted thereinto and a production method of the engineered antibody.

The method of producing an engineered antibody according to the present invention is a method of producing an engineered antibody, including:
selecting, as a cyclic peptide, a cyclic peptide having a binding ability to a second target molecule different from a first target molecule to be recognized by an antibody to be inserted, preferably selecting a cyclic peptide available by a display type finding system,
selecting, from the amino acid sequences of the thus-selected cyclic peptide, an internal amino acid sequence to be inserted in the antibody and selecting a base sequence corresponding to the internal amino acid sequence,
selecting two amino acid residues, in the Fc region of the antibody, between which the internal amino acid sequence is inserted,
selecting a base sequence corresponding to the amino acid sequence in the Fc region of the antibody, deleting, if necessary, a base sequence corresponding to the amino acid residue present between the two amino acid residues, and inserting and incorporating the selected base sequence corresponding to the internal amino acid sequence of the cyclic peptide to prepare a nucleic acid having the thus-incorporated base sequence, and
translating the nucleic acid.

When the selected base sequence corresponding to the internal amino acid sequence of the cyclic peptide is inserted and incorporated to obtain the thus-incorporated base sequence, a base sequence corresponding to a linker may be inserted and incorporated.

As the cyclic peptide, a cyclic peptide selected by the mRNA display method such as RaPID or TRAP or the phage display method is preferred, with that selected by the mRNA display method being more preferred. The base sequence of the internal amino acid sequence of the cyclic peptide to be inserted can be easily understood according to the mRNA display method or the like.

A method of selecting base sequences corresponding to two amino acid sequences present in a site, in the Fc region of an antibody, to be inserted with a cyclic peptide and deleting, if necessary, a base present between the selected base sequences or a method of inserting the base sequence corresponding to the internal amino acid sequence of the selected cyclic peptide to prepare a nucleic acid having the thus-incorporated base sequence (including a base sequence corresponding to a linker, if desired) is not particularly limited and can be performed based on a conventionally known method.

A method of translating the nucleic acid thus prepared is a known matter in the technical field to which the present invention belongs so that translation of the nucleic acid may be performed using such known methods as needed.

The engineered antibody obtained by the present invention has, inserted in the Fc region thereof, an internal peptide sequence of a cyclic peptide having a binding ability to a second target molecule different from a first target molecule to be recognized by the antibody and therefore having a binding ability to the first and second target molecules.

The engineered antibody preferably binds to the first and second target molecules simultaneously. When it simultaneously binds to the first and second target molecules and the first and second target molecules are respectively different cell surface molecules, the engineered antibody of the present invention can induce adhesion of heterologous cells via a linkage between these two respectively-different cell surface molecules.

In the present invention, the engineered antibody of the present invention obtained using, for example, an antigen of cancer cells as a first target and an antigen on T lymphocytes or NK cells as a second target is capable of inducing immune attack against the cancer cells and effectively and specifically damaging and killing them. In addition, for example, for allowing a therapeutic antibody to pass through the blood-brain barrier and delivering it into the brain, a molecule (transferrin receptor or the like) which has expressed in the brain blood vessels may be used as the second target.

The present invention has following characteristics:
(1) not limiting the IgG type or antigen specificity of an original existing antibody (that defines the first binding specificity),
(2) not including a step of selecting based on a partial random-sequence library of the antibody itself,
(3) capable of manufacturing an engineered antibody, that is, a final product on an equal efficiency level with that of an original wild type antibody,
(4) capable of using any protein as a target of multispecificity insofar as it is an object from which a cyclic peptide binding with high affinity by the RaPID method or the like can be obtained, and
(5) capable of simultaneously granting at most 9 independent additional binding specificities to an existing antibody.

### Examples

The present invention will hereinafter be described specifically by Examples, but the present invention is not limited to or by the following Examples.

Cyclic peptides having high affinity for a target protein were obtained by performing the RaPID system based on WO2011/049157 and Japanese Patent Application Laid-Open No. 2013-46637. Cyclic peptides binding specifically to human-derived receptors plexin B1, Met, EGF receptor, and TrkB, respectively, were used. The structure of the cyclic peptides are shown in Fig. 1

In Fig. 1, the sequence of the variable region derived from each of the cyclic peptides was inserted in the Fc region of an antibody. D-amino acids indicated by small letters w and y as the N-terminal amino acids of the amino acid sequence were introduced into the Fc region after substitution by L-amino acids or deletion. When they were inserted, a linker sequence composed of 0 to 4 residues was added while sandwiching the above-described sequence of the variable region therewith.

### [Example 1] Addition of plexin B1 binding specificity to neuropilin-1 antibody

### 1. Design of peptide insertion

In order to present the internal sequence of a cyclic peptide on the Fc region of an antibody while keeping a target binding activity, an insertion site was searched with reference to the tertiary structure of the Fc region of the antibody. Nine loop portions exposed on the molecular surface and linking secondary structural portions were selected from the Fc region. The loop portions thus selected are shown in Fig. 2. The selected loops include three (T1 to 3) at Top near the hinge of the Fc region, three (M1 to 3) at Middle, and three (B1 to 3) at Bottom. All of their positions on the full Fc amino acid sequence are shown in Fig. 3.

### 2. Preparation of bispecific antibody

An N1 antibody (another name of clone: YW64.3) against neuropilin 1 was used as an antibody to be inserted with a peptide (Liang et al. J. Mol. Biol. 2007). The heavy chain variable region of the N1 antibody was linked with a DNA encoding the human IgG1 heavy chain constant region, followed by incorporation in an expression vector p3XFLAG-CMV-14 (product of Sigma Aldrich). As for the light chain, a DNA encoding the full length of it was incorporated also in a p3XFLAG-CMV-14 vector.

Based on the N1 heavy chain expression vector, expression vectors having an mP6-9 peptide sequence inserted in the vicinity (*) of the center of nine loop structures in the Fc region shown in Fig. 3 were prepared. The inserted sequence portion was amplified using the extension PCR method. The peptide-inserted antibodies thus obtained were each described in the following manner: "name of antibody (name of cyclic peptide_insertion site)" such as N1 IgG(mP6-9_T1).

Expi293F cells (product of Thermo Fisher Scientific) were seeded in 3 mL of Expi293 Expression Medium (product of Thermo Fisher Scientific) to give 3×10⁶ cells/mL. Then, gene introduction, into the Expi293F cells, of the respective expression vectors of the antibody light chain and the antibody heavy chain (or inserted products thereof), each 1.5 µg, was performed in a conventional manner by using ExpiFectamine 293 Reagent (product of Thermo Fisher Scientific).

The gene introduction was followed by shaking culture of the cells at 125 rpm for 18 hours under conditions of 37°C and 8% CO₂. Then, 15 µL and 150 µL of ExpiFectamime 293 Transfection Enhance er 1 and ExpiFectamime 293 Transfection Enhancer 2 (product of Thermo Fisher Scientific) were added, respectively followed by shaking culture at 125 rpm for 3 days under conditions of 37°C and 8% CO₂ to collect a culture supernatant.

Protein A-Sepharose (30 µL, product of Thermo Fisher Scientific) was added to 0.3 mL of the culture supernatant thus collected and the resulting mixture was mixed by rotation for 2 hours. The Sepharose was precipitated by centrifugal separation to remove the supernatant and washed three times with 1 mL of Tris-buffered saline (TBS, 20 mM Tris-HCI, 150 mM NaCl, pH 7.5). After addition of 20 µL of an SDS sample buffer, the resulting mixture was heated at 95°C for 2 minutes to elute a sample. The sample thus eluted (5 µL) was subjected to electrophoresis under reducing conditions and stained with Coomassie brilliant blue.

The results of electrophoresis are shown in Fig. 4. In Fig. 4, samples are indicated by a circled number. The band of N1 IgG (Sample No. 1) is observed at molecular weights (50 kDa and 25 kDa) where bands corresponding to the heavy chain and the light chain are expected to exist and the respective heavy chains of all the peptide-inserted antibodies (Sample Nos. 2 to 10) exhibit a molecular weight slightly higher than that. This agrees with the overall amino acid length longer by 16 to 17 residues. It has been confirmed that the inserted antibodies are each expressed·secreted from the Expi293F cells in an amount comparable to that of N1 IgG having no peptide inserted therein.

### 3. Addition of plexin B1 binding ability to neuropilin-1 antibody

A binding test by flow cytometry was performed to examine whether or not the respective N1 IgGs having a mP6-9 peptide inserted at various positions on the Fc thereof had a binding ability to plexin B1 which the cyclic mP6-9 peptide originally had had. The respective expression supernatants of N1 IgG and nine mP6-9 peptide inserted products were caused to react with stable plexin B1-expressing cells (described in Cell Chem. Biol, 2016, 23, 1341-1350). The IgGs thus bound were stained with an Alexa Fluor 488-labeled goat anti-human IgG secondary antibody (product of Thermo Fischer) and analyzed by EC800 flow cytometer (product of Sony).

The results of the flow cytometry are shown in Fig. 5. The un-engineered N1 antibody showed only a background level of binding to the stable plexin B1-expressing cells (panel 1, shown by a circled number in the drawing), while all the N1 antibodies having mP6-9 inserted therein showed 1.5- to 10-fold binding, suggesting that these antibodies all newly acquired a binding specificity to plexin B1 in addition to that to neuropilin 1.

### [Example 2] Addition of various binding specificities to neuropilin-1 antibody

### 1. Design of peptide insertion and preparation of antibody

By using expression vectors obtained by adopting the "B1 group" shown in Example 1 as a peptide insertion position and inserting the internal sequences of the four kinds of cyclic peptides shown in Fig. 1 according to the method of Example 1, preparation of recombinant antibodies and expression evaluation of them were performed also as in Example 1.

The results of electrophoresis are shown in Fig. 6. In Fig. 6, samples are indicated by a circled number. The band of N1 IgG (Sample No. 1) is observed at the molecular weights (50 kDa and 25 kDa) where bands corresponding to the heavy chain and the light chain are expected to exist and the respective heavy chains of all the peptide-inserted antibodies (Sample Nos. 2 to 10) exhibit a molecular weight slightly higher than that of N1 IgG (Sample No. 1). This agrees with the overall amino acid length longer by 17 to 21 residues. It has been confirmed that the cycle-inserted antibodies are each expressed·secreted from the Expi293F cells in an amount comparable to that of N1 IgG having no peptide inserted therein.

### 2. Addition of various binding abilities to neuropilin-1 antibody

A binding test by flow cytometry was performed to examine whether or not the respective N1 IgGs having various cyclic peptides inserted therein retained the molecule binding ability which the original cyclic peptides had had. The expression supernatants of the respective N1 IgGs having four kinds of peptides inserted therein were caused to react with HEK293 cells (mock cell) as a control or cells expressing various receptors and the IgGs thus bound were stained with an Alexa Fluor 488-labeled goat anti-human IgG secondary antibody (product of Thermo Fisher) and analyzed by an EC800 type flow cytometer (product of Sony Corporation).

The results of the flow cytometry are shown in Fig. 7. Receptor expression cells used therefor are stable plexin B1-expressing cells (panel 1, indicated by a circled number in the drawing), stable Met-expressing cells (panel 2), transient EGFR-expressing cells (panel 3), and transient TrkB expressing cells (panel 4). All the peptide-inserted N1 antibodies showed clearly enhanced binding histograms to the target molecule-expressing cells compared with the control cells, suggesting that the peptide insertion enables to grant the binding property of the cyclic peptide binder to the neuropilin-1 antibody.

### [Example 3] Addition of various binding specificities to various antibodies 1. Preparation of bispecific antibody

Peptide-inserted bispecific antibodies were prepared, respectively, using, as well as the neuropilin-1 antibody used in Examples 1 and 2, two human IgG1 antibodies (human PD-L1 antibody Avelumab and human CD3 antibody OKT3) and one mouse IgG1 antibody (mouse transferrin receptor antibody 8D3). As the variable region amino acid sequence of Avelumab, OKT3, and 8D3, those described in WO2013079174A1, Arakawa et al, J Biocheme 1996, and Boado et al, Biotech Bioeng. 2008 were used, respectively. DNAs corresponding thereto were synthesized and the heavy chain and the light chain were incorporated in an expression vector p3XFLAG-CMV-14 (product of Sigma Aldrich) as in Example 1. For 8D3, however, the heavy chain and light chain constant regions derived from Mouse IgG1 were used.

2. Evaluation of bispecific antibody using neuropilin-1 antibody Recombinant expression of respective antibodies obtained by inserting a Met binding peptide sequence (aMD5) and an EGF receptor binding sequence (A6-2f) into the B1 loop of N1 IgG was performed by a method shown in Example 2. The results of electrophoresis are shown in Fig. 8A. The resulting peptide-inserted antibodies exhibited an expression efficiency on the same level with that of the non-inserted N1 antibody. Binding of them to target molecules were evaluated by flow cytometry similar to that of Example 2.

The results of flow cytometry are shown in Fig. 8B. The results show that binding to neuropilin 1, that is, first binding specificity of the N1 antibodies was observed (panel of second row) equally in HEK cells in which neuropilin 1 was transiently expressed, showing that peptide insertion does not damage the original binding property. Further, mutant antibodies having the aMD5 or A6-2f peptide inserted into the B1 loop thereof exhibit a strong positive signal in the Met (panel of third row) and EGF receptor expressing cells (panel of fourth row), from which construction of a bispecific antibody can be confirmed.

### 3. Evaluation of bispecific antibody using PD-L1 antibody Avelumab

The recombinant expression of respective antibodies obtained by inserting a plexin B1 binding peptide sequence (mP6-9), a Met binding peptide sequence (aMD5), and an EGF receptor binding sequence (A6-2f) into the B1 or B2 loop of the Avelumab IgG was performed in a manner similar to that of Example 2. The results of electrophoresis are shown in Fig. 9A. The resulting peptide-inserted antibodies exhibited an expression efficiency on the same level with that of the non-inserted Avelumab antibody. Binding of them to target molecules was evaluated by flow cytometry similar to that of Example 2.

The results of flow cytometry are shown in Fig. 9B. The results show that binding to PD-L1, that is, first binding specificity of the Avelumab antibodies was observed (panel of second row) equally in a cell line (human breast cancer cells MDA-MB-321) constantly expressing PD-L1, showing that peptide insertion does not damage the original binding property. Further, mutant antibodies obtained by inserting aMD5 or A6-2f peptide into the B1 or B2 loop exhibit a strong positive signal in the stable Plexin B1 expressing cells (panel of third row), stable Met expressing cells (panel of fourth row) and transient EGF receptor expressing cells (panel of fifth row), from which construction of a bispecific antibody can be confirmed.

### 4. Evaluation of bispecific antibody using CD3 antibody OKT3

The recombinant expression of respective antibodies obtained by inserting a plexin B1 binding peptide sequence (mP6-9) or a Met binding peptide sequence (aMD4) into the B2 loop of OKT3 IgG was performed in a manner similar to that shown in Example 2. The results of electrophoresis are shown in Fig. 10A. The resulting peptide-inserted antibodies exhibited an expression efficiency on the same level with that of the non-inserted OKT3 antibody. Binding of them to target molecules was evaluated by flow cytometry similar to that of Example 2.

The results of flow cytometry are shown in Fig. 10B. The results show that binding to human CD3, that is, first binding specificity of the OKT3 antibodies was observed (panel of second row) equally in a cell line (Jurkat cells) constantly expressing CD3, showing that peptide insertion does not damage the original binding property. Further, mutant antibodies having the mP6-9 or aMD4 peptide inserted into the B2 loop thereof exhibit a strong positive signal in the stable Plexin B1 expressing cells (panel of third row) and stable Met expressing cells (fourth panel, from which construction of a bispecific antibody can be confirmed.

5. Evaluation of bispecific antibody using transferrin receptor antibody 8D3

After specifying the loop positions corresponding to B1 to B3 in the mouse heavy chain of a 8D3 antibody as mouse IgG1 with reference to Fig. 3 and preparing expression vectors having a plexin B1 binding peptide sequence (mP6-9) inserted by a method similar to that of Example 1, the resulting vectors and also a light chain (mouse kappa chain) expression vector were transfected into Expi293F cells. The recombinant antibodies expressed and secreted in the supernatant was precipitated using protein G-Sepharose (product of Thermo Fisher Scientific) and analyzed by electrophoresis.

The results of electrophoresis are shown in Fig. 11A. The peptide-inserted antibodies exhibited an expression efficiency on the same level with that of the non-inserted 8D3 antibody. A binding test by flow cytometry was performed in order to evaluate binding of them to a target molecule. Expression supernatants of 8D3 IgG and three kinds of peptide-inserted antibodies were caused to react with cells expressing various receptors and the mouse IgG thus bound was stained with an Alexa Fluor 488-labeled goat anti-mouse IgG secondary antibody (product of Thermo Fisher) and analyzed using an EC800 flow cytometer (product of Sony).

The results of flow cytometry are shown in Fig. 11B. The results show that binding to a mouse transferrin receptor, that is, first binding specificity of the 8D3 antibodies was observed (panel of second row) equally in cells (TfR cells) that transiently expressed TfR, showing that peptide insertion does not damage the original binding property. Further, mutant antibodies having the mP6-9 peptide inserted into the B1 to B3 loops thereof exhibit a strong positive signal in the stable Plexin B1 expressing cells (panel of third row), from which construction of a bispecific antibody can be confirmed.

### 6. Evaluation of multispecific antibody using neuropilin-1 antibody

Addition of various specificities was so far tried individually. Here, activity of one antibody simultaneously granting two or more specificities is evaluated. The recombinant expression of respective antibodies obtained by using N1 IgG and inserting various combinations of a plexin B1 binding peptide sequence (mP6-9), a Met binding peptide sequence (aMD4), and an EGF receptor binding sequence (A6-2f) into various loops of the antibody was performed in a manner similar to that of Example 2. Binding of them to target molecules was evaluated by flow cytometry as described above up to 5.

The results of flow cytometry are shown in Fig. 12. The results show that binding to neuropilin 1, that is, first binding specificity of the N1 antibodies was observed (panel of second row) equally in HEK cells in which neuropilin 1 was transiently expressed, showing again that peptide insertion does not damage the original binding property. Further, respective mutant antibodies having mP6-9, aMD4 and A6-2f peptides inserted therein exhibit a strong positive signal in the HEK cells in which Plexin B1 (panel of third row), Met (panel of fourth row), and EGF receptor (panel of fifth row) were transiently expressed and their binding did not depend on the insertion site or presence of another peptide inserted simultaneously. From the results, it can be confirmed that these engineered antibodies have acquired, in addition to the original single antigen specificity, bispecificity when one kind of a peptide is inserted, trispecificity when two kinds of peptides are inserted, and quad-specificity when three kinds of peptides are inserted.

### [Example 4] Confirmation of simultaneous double-target binding ability of peptide-inserted IgG

### 1. Principle of sandwich type intermolecular crosslinking test and preparation of target antigen

Although evaluation of each of the first and the second binding specificities of the peptide inserted IgG was described above up to Example 3, a sandwich ELISA type assay system was constructed to show that the peptide inserted IgG had ability of simultaneously binding to two targets. The principle is shown in Fig. 13A. First, based on the method described in Protein Exp. Purification, 2014, 95, 240-247, a construct encoding a fusion protein having a PA tag (product of Wako Pure Chemical Industries) added to the C terminal of the extracellular region of three kinds of second target antigens (each, single-transmembrane receptor) and the resulting construct was incorporated in an expression vector pcDNA 3.1 (product of Thermo Fisher Scientific). Transient expression was caused in Expi293F cells by using the resulting vector according to a method similar to that of Example 1 to prepare respective culture supernatants containing Plexin B1-PA, Met-PA, and EGFR-PA, each soluble receptor fragment and they were purified with Protein A Sepharose. On the side of the first antigen, pAPtag-5 (product of GenHunter), an AP fusion expression vector, was used to fuse the sequence of the extracellular region thereof with the N terminal (neuropilin 1 and PD-L1) of AP or the C terminal (transferrin receptor), followed by transient expression in Expi293F cells in a manner similar to that of Example 1 to prepare culture supernatants containing Nrp-1ec-AP, PD-L1ec-AP, and AP-TfRec, each a soluble receptor-AP fusion product, respectively.

### 2. Sandwich type binding test

Crosslinking between target molecules by the peptide inserted IgG was evaluated according to the following protocol based on the principal shown in Fig. 13A.
(1) A purified second-target antigen solution (50 µL) diluted to 10 µg/mL was added to a 96-well plate and allowed to stand at 4°C for 16 hours.
(2) After suction by an aspirator, 200 µL/well of a 5% skim milk in Tris-buffered saline (TBS; 20 mM Tris-HCI, 150 mM NaCl, pH 7.5) was added and the plate was allowed to stand at room temperature for one hour.
(3) Various Addbody expression supernatants (50 µL) were added and the plate was allowed to stand at room temperature for one hour.
(4) The plate was washed three times with 200 µL/well of TBS.
(5) A supernatant expressing an AP fusion first target antigen (50 µL) was added and the plate was allowed to stand at room temperature for 30 minutes.
(6) The plate was washed four times with 200 µL/well of TBS.
(7) A chromogenic substrate (100 µL/well) (Phosphatase substrate, product of Sigma Aldrich) was added and after the plate was allowed to stand at room temperature for 5 to 60 minutes, the absorbance at 405 nm of the solution in each well was measured.

The results are shown in Fig. 13B. In any of the neuropilin antibody N1, PD-L1 antibody Avelumab, and transferrin receptor antibody 8D3, the peptide inserted IgG was captured on the plate having their second target antigens immobilized thereon and bound to a first target antigen-AP fusion protein added later to allow color development of the substrate (gray bar). On the other hand, the plain IgG having no peptide inserted therein did not give a positive signal (black bar). This suggests that each Addbody has a binding ability to the first and second targets not individually but simultaneously.

### [Example 5] Induction of adhesion between heterologous cells by peptide inserted IgG

### 1. Principle of test on adhesion between heterologous cells and preparation of cells

When the first and second target molecules of a peptide inserted IgG are receptors present on respectively different cells, the peptide inserted IgG is expected to be effective for inducing approximation and adhesion of these heterologous cells. In order to examine this effect, an experiment was performed based on the principle as shown in Fig. 14A by using OKT3 (aMD4_B2) obtained by inserting an aMD4 peptide sequence, a Met binder, into the B2 loop of OKT3, a CD3 antibody. Human acute T-cell leukemia-derived cell line Jurkat was used as CD3 expressing cells and Chinese hamster ovarian cells (Met-CHO cells) stably exhibiting high expression of a human Met receptor was used as Met expressing cells. The Met-CHO cells were labeled with a red fluorescent dye DiD (product of Biotium), seeded on a 96-well plate, and cultured for 3 hours in a 10% serum-containing medium to cause adhesion. The Jurkat cells were labeled with a green fluorescent dye Neuro DiO (product of Biotium), were caused to react for 30 minutes on ice with a wild type OKT3 or peptide-inserted OKT3 (aMD4_B2) expression supernatant prepared in a manner similar to that of Example 3, and after washing once, were seeded on the Met-CHO cells. After culturing for 17 hours in a serum-free medium containing 1 mg/ml of BSA, washing was performed twice with 200 µL /well of a serum-free medium and a fluorescent image and a phase difference image were recorded using a digital fluorescence microscope (BZ-X700, product of Keyence).

The image of the results are shown in Fig. 14B. The Met-CHO cells which have adhered to the plate and Jurkat cells which have adhered onto the plate or Met-CHO cells are visualized on the red fluorescent image (excitation wavelength: 620 nm, fluorescent wavelength: 700 nm) and on the green fluorescent image (excitation wavelength: 470 nm, fluorescent wavelength: 525 nm), respectively. These images are overlaid and the adhesion site of the heterologous cells (meaning the site where red and green cells have overlapped with each other) is marked with a white blank circle. Totally ten visual fields are selected from a plurality of wells and the number of the Jurkat cells which have adhered to the Met-CHO cells is quantified into a graph and shown in Fig. 14C. This shows that the peptide-inserted OKT3 simultaneously recognizes the Met molecule on the Met-CHO cells and the CD3 molecule on the Jurkat cells and crosslinks between these cells by its interaction.

## Claims

1. A method of granting a binding ability to a second target molecule to an antibody, comprising inserting an internal peptide sequence of a cyclic peptide into an Fc region of the antibody, wherein the cyclic peptide has the binding ability to the second target molecule different from a first target molecule to be recognized by the antibody.

2. The method according to Claim 1, wherein the Fc region of the antibody is derived from human, mouse, rat, rabbit, horse, or dog.

3. The method according to Claim 1 or 2, wherein a site into which the internal peptide sequence is inserted is a loop portion exposed on a molecular surface of the Fc region.

4. The method according to any one of Claims 1 to 3, wherein the cyclic peptide is a cyclic peptide obtained by a display type finding system.

5. An engineered antibody having a binding ability to a first target molecule and a second target molecule, obtained by inserting an internal peptide sequence of a cyclic peptide into an Fc region of an antibody, wherein the cyclic peptide has the binding ability to the second target molecule different from the first target molecule to be recognized by the antibody.

6. The engineered antibody according to Claim 5, wherein the engineered antibody simultaneously binds to the first and second target molecules.

7. The engineered antibody according to Claim 5 or 6, wherein the first and second target molecules are respectively different cell surface molecules and the engineered antibody induces adhesion between heterologous cells via binding with two different cell surface molecules.


SEQUENCE LISTING

<110>  The University of Tokyo
       Osaka University

<120>  A highly versatile method for granting new binding specificity to
        antibody

<130>  T0529AKP0012

<150>  JP2018-144345
<151>  2018-07-31

<160>  16

<170>  PatentIn version 3.5

<210>  1
<211>  13
<212>  PRT
<213>  Artificial Sequence

<220>
<223>  variable region of cyclic peptide mP6-9

<400>  1

Arg Pro Tyr Ile Glu Arg Trp Thr Gly Arg Leu Ile Val
1               5                   10


<210>  2
<211>  14
<212>  PRT
<213>  Artificial Sequence

<220>
<223>  variable region of cyclic peptide aMD4

<400>  2

Arg Gln Phe Asn Arg Arg Thr His Glu Val Trp Asn Leu Asp
1               5                   10


<210>  3
<211>  13
<212>  PRT
<213>  Artificial Sequence

<220>
<223>  variable region of cyclic peptide aMD5

<400>  3

Trp Tyr Tyr Ala Trp Asp Gln Thr Tyr Lys Ala Phe Pro
1               5                   10


<210>  4
<211>  15
<212>  PRT
<213>  Artificial Sequence

<220>
<223>  variable region of cyclic peptide A6-2f

<400>  4

Ala Ser Phe Pro Glu Glu Leu Asp Glu Trp Tyr Val Ala Tyr Gly
1               5                   10                  15


<210>  5
<211>  11
<212>  PRT
<213>  Artificial Sequence

<220>
<223>  variable region of cyclic peptide trkD5

<400>  5

Arg Gln Ala Ser Pro Lys Phe Thr Phe Trp Ser
1               5                   10


<210>  6
<211>  227
<212>  PRT
<213>  Homo sapiens

<400>  6

Asp Lys Thr His Thr Cys Pro Pro Cys Pro Ala Pro Glu Leu Leu Gly
1               5                   10                  15


Gly Pro Ser Val Phe Leu Phe Pro Pro Lys Pro Lys Asp Thr Leu Met
            20                  25                  30


Ile Ser Arg Thr Pro Glu Val Thr Cys Val Val Val Asp Val Ser His
        35                  40                  45


Glu Asp Pro Glu Val Lys Phe Asn Trp Tyr Val Asp Gly Val Glu Val
    50                  55                  60


His Asn Ala Lys Thr Lys Pro Arg Glu Glu Gln Tyr Asn Ser Thr Tyr
65                  70                  75                  80


Arg Val Val Ser Val Leu Thr Val Leu His Gln Asp Trp Leu Asn Gly
                85                  90                  95


Lys Glu Tyr Lys Cys Lys Val Ser Asn Lys Ala Leu Pro Ala Pro Ile
            100                 105                 110


Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210>  7
<211>  208
<212>  PRT
<213>  Homo sapiens

<400>  7

Val Phe Leu Phe Pro Pro Lys Pro Lys Asp Thr Leu Met Ile Ser Arg
1               5                   10                  15

Thr Pro Glu Val Thr Cys Val Val Val Asp Val Ser His Glu Asp Pro
            20                  25                  30

Glu Val Lys Phe Asn Trp Tyr Val Asp Gly Val Glu Val His Asn Ala
        35                  40                  45

Lys Thr Lys Pro Arg Glu Glu Gln Tyr Asn Ser Thr Tyr Arg Val Val
    50                  55                  60

Ser Val Leu Thr Val Leu His Gln Asp Trp Leu Asn Gly Lys Glu Tyr
65                  70                  75                  80

Lys Cys Lys Val Ser Asn Lys Ala Leu Pro Ala Pro Ile Glu Lys Thr
                85                  90                  95

Ile Ser Lys Ala Lys Gly Gln Pro Arg Glu Pro Gln Val Tyr Thr Leu
            100                 105                 110

Pro Pro Ser Arg Asp Glu Leu Thr Lys Asn Gln Val Ser Leu Thr Cys
        115                 120                 125

Leu Val Lys Gly Phe Tyr Pro Ser Asp Ile Ala Val Glu Trp Glu Ser
    130                 135                 140

Asn Gly Gln Pro Glu Asn Asn Tyr Lys Thr Thr Pro Pro Val Leu Asp
145                 150                 155                 160

Ser Asp Gly Ser Phe Phe Leu Tyr Ser Lys Leu Thr Val Asp Lys Ser
                165                 170                 175

Arg Trp Gln Gln Gly Asn Val Phe Ser Cys Ser Val Met His Glu Ala
            180                 185                 190

Leu His Asn His Tyr Thr Gln Lys Ser Leu Ser Leu Ser Pro Gly Lys
        195                 200                 205

<210>  8
<211>  5
<212>  PRT
<213>  Homo sapiens

<400>  8

Ser His Glu Asp Pro
1               5

<210>  9
<211>  4
<212>  PRT
<213>  Homo sapiens

<400>  9

Tyr Asn Ser Thr
1

<210>  10
<211>  7
<212>  PRT
<213>  Homo sapiens

<400>  10

Asn Lys Ala Leu Pro Ala Pro
1               5

<210>  11
<211>  4
<212>  PRT
<213>  Homo sapiens

<400>  11

Val Asp Gly Val
1

<210> 12
<211> 3
<212> PRT
<213> Homo sapiens

<400> 12

Lys Gly Gln
1

<210> 13
<211> 4
<212> PRT
<213> Homo sapiens

<400> 13

Ser Asp Gly Ser
1

<210> 14
<211> 4
<212> PRT
<213> Homo sapiens

<400> 14

Thr Lys Asn Gln
1

<210> 15
<211> 3
<212> PRT
<213> Homo sapiens

<400> 15

Ser Asn Gly
1

<210> 16
<211> 5
<212> PRT
<213> Homo sapiens

<400> 16

Gln Gln Gly Asn Val
1               5